# EUROPEAN PATENT SPECIFICATION

(11) **EP 4 574 062 B1**
(45) Date of publication and mention of the grant of the patent: **04.03.2026**
(21) Application number: 23219221.1
(22) Date of filing: 21.12.2023
(51) Int. Cl.: A61B 17/225, A61B 17/22, A61H 23/00

(54) **DEVICE FOR GENERATING SHOCKWAVES AND METHOD OF MANUFACTURING THEREOF**
VORRICHTUNG ZUR ERZEUGUNG VON STOSSWELLEN UND VERFAHREN ZU DEREN HERSTELLUNG
DISPOSITIF DE GÉNÉRATION D'ONDES DE CHOC ET SON PROCÉDÉ DE FABRICATION

(43) Date of publication of application: 25.06.2025
(73) Proprietor: Heart Regeneration Technologies GmbH, 6020 Innsbruck (AT)
(72) Inventor: DORFMÜLLER, Christian, 78239 Rielasingen (DE); HOLFELD, Johannes, 2380 Perchtoldsdorf (AT)
(74) Representative: Vossius & Partner Patentanwälte Rechtsanwälte mbB

(56) References cited:
- DE-A1- 102021 203 544
- US-A1- 2007 016 112
- US-A1- 2010 022 944

## Description

The present invention relates to a device for generating shockwaves and a method of manufacturing a device for generating shockwaves.

For several decades, shockwaves at low-energy levels have been known, mainly in sports medicine, orthopedics and wound healing. The first human medical application of high-energy extracorporeal shockwaves was used in the field of urology using its mechanical characteristics in liquids to destroy urinary concernments within the patient's body. For example, such shockwaves are used to destroy kidney stones. This medical procedure is known as lithotripsy. A further application of shockwaves is Direct Epicardial Shock Wave Treatment, DESWT. DESWT induces tissue regeneration in infarcted heart muscle and thereby significantly improves and restores heart function (i.e., left ventricular ejection fraction). The shockwaves initiate biological responses like new heart muscle tissue formation.

Shockwaves in the above applications may be generated electro-hydraulically. Electrohydraulic shockwave generation means that a liquid is held in a closed chamber with at least two electrodes inside. A high voltage is applied between the two electrodes, for example more than 1 kV. Upon an electrical discharge between the two electrodes, a part of the liquid is vaporized by a plasma generated by the electrical discharge between the electrodes. This causes an acoustic shock wave pressure pulse in the liquid (typically water) which is then transferred via a membrane to the patient's skin or tissue. Some shockwave generators additionally include a reflector for focusing the shockwave's energy to a certain point or a geometrical form like a ring or a line, depending on the designated treatment area within the patient's body. For example, an ellipsoidal reflector may focus a point like shockwave generated between the tips of the electrodes in the first focal point into a second focal point. The second focal point is typically positioned within the patient's body and defined as the treatment area. Alternatively, a parabolic reflector may generate flat unfocused parallel shockwave pressure fronts like in the treatment of skin burns and other superficially occurring treatment indications. If there is no reflector, the shockwaves radiate spherically from the electrical discharge between the electrode tips. These unfocused shock waves are used in the treatment of arteriosclerosis within coronary arteries by electrodes inserted via a coronary catheter.

Many of the above medical indications can be treated by using a small handheld shock wave applicator (shock wave probe). While the shockwaves have therapeutic benefits, they present a challenge from a design perspective. Some of the energy is transferred and/or absorbed by a housing of the probe and the components contained therein. In particular, vibrations in the applicator caused by the shockwaves may over time loosen components, damage components, and require maintenance of the shockwave applicator. For example, the shockwaves and corresponding vibrations may damage electronic components, may loosen screws, and may damage an isolation of high voltage parts.

US 2007/0016112 A1 shows a system for treating an internal organ having a generator source for producing a shock wave connected to a portable shock wave applicator device. The shock wave applicator device has a side-firing shock wave head having a variable angle adjustment relative to a release and lock connected handle or holder means for holding said device. The inclination of the shock wave head can be set to a fixed inclination to reach the organ at various locations or surfaces or can be pivotally inclined continuous to vary the treatment surfaces area.

US 2010/022944 A1 shows a method and apparatus for treating an acute myocardial infarction. The apparatus comprises an ultrasound catheter that can be inserted into the esophagus to deliver ultrasound energy to the heart and coronary vasculature. The ultrasound energy can enhance the effect of an intravenously administered thrombolytic drug on a blood clot within the coronary vasculature. The ultrasound catheter can have a means for promoting acoustic coupling with the esophagus and a means for preventing or reducing aspiration of fluids into the lungs.

DE102021203544 A1 shows an electroacoustic converter intended for generating sound waves, in particular shock waves, for treating the human or animal body. It has piezoelectric elements which are arranged inside a carrier in a housing. A free space is formed between the piezoelectric elements and the housing and is filled with a casting compound.

The present disclosure addresses one or more of the above-mentioned disadvantages. In particular, the present disclosure has the object of providing a device for generating shockwaves that is durable and reliable.

A first aspect of the present invention relates to a medical device for generating shockwaves. The device comprises a housing. The housing encompasses a pressure pulse source with a chamber housing defining a chamber and a shockwave opening. The chamber is configured to be filled with a liquid. A plurality of electrodes is disposed in the chamber and configured to be coupled to an electrical pulse generating system. The plurality of electrodes include a first electrode and a second electrode. The first electrode and the second electrode define a spark gap. According to the present disclosure, at least a part of an interior of the housing is potted with a potting compound configured to absorb vibrations caused by the generated shockwaves.

Potting at least a part of the interior of the housing secures components held within the housing securely in place. It is a simple process, which provides for a reliable and durable fixation of components in the housing. This improves a longevity of the device and its resistance to vibrations caused by the generated shockwaves. In contrast to the known approaches, which rely on improving a fixation of each and every component, the presently proposed potting allows for a simpler fixation technique of the potted components.

The device may be sterilizable using steam, Gamma rays, plasma, ethylene oxide (ETO) and/or formaldehyde. The potting compound protects components held inside the housing during sterilization. Sterilization may involve the application of steam at elevated temperatures which may be corrosive to components held within the device, e.g., the pressure pulse source, the chamber, the plurality of electrodes and/or any other mechanical or electrical parts. Sterilization may also involve Gamma, plasma, and ETO (ethylene oxide or formaldehyde) sterilization.

The potting compound may be electrically insulating. This may reduce electrical leakage and leakage currents which are critical for especially Direct Epicardial Shock Wave Treatment, DESWT. The potting may protect the interior components when the device is dropped or otherwise exposed to high forces. Even if part of the housing is damaged the potted internal parts of the device are secured and optionally electrically isolated.

Further advantages of the potting may be an improved heat dissipation, corrosion protection and a protection against chemicals and environmental influences.

Further, the potting compound prevents an extreme temperature increase on the surface of the handheld device, which is due to the high power dissipation by electrohydraulic shock wave formation process.

In addition, the potting compound may form a thermal barrier or thermally insulating layer. If heat develops in the pressure pulse source or elsewhere inside the housing, then a user is protected from the heat.

The components might be in a form before the process of potting in different components as solid materials, granulate, powder, liquid, or gels. The final potting compound may be an elastic material with different and selected or adjusted shore hardness and elastic characteristic of its material modules. The shore hardness of the potting compound (after potting) may be 0 to 60 Shore A, in particular 10 to 50 Shore A, and preferably 20 to 40 Shore A. A most preferred range is 28 to 34 Shore A. These hardness's provide a particularly good mechanical protection, e.g. in the case the device falls.

A pressure pulse or shockwave may be an acoustic pulse which includes several cycles of positive and negative pressure. The amplitude of the positive part of such a cycle may be 0.1 MPa to 100 MPa. Preferably, the range is between 10 and 100 MPa. A time duration of a pressure pulse or shockwave may be below a microsecond to about a second. Preferably, the duration is from 0.5 µs to 10 µs.

The chamber housing defines a chamber in which the shock wave is generated by a plasma discharge between the multiple electrodes in a liquid. Additionally, the pressure pulse source may comprise a membrane. The membrane may cover the shockwave transmission opening. Together, the membrane and the chamber housing may define a closed chamber for shockwave generation. Preferably, the potting compound fixates the membrane. This may mean the potting compound may fixate a position of the membrane, e.g., by attaching a radial outer edge of the membrane to the chamber housing.

The closed chamber is configured to be filled with a liquid. Preferably, the closed chamber comprises the liquid, and is advantageously filled with the liquid. The liquid may be water. The term liquid may include any liquid substances including mixtures, solutions, suspensions, and dispersions. The liquid may additionally comprise additive substances or materials. The additive substances or materials may, e.g., change the conductivity of the liquid. The conductivity and filling (e.g., pressure) of the liquid influences the underwater spark discharge characteristics and therefore the lag times and rise times and intensity of the electro-hydraulic shockwaves that are generated within the chamber. Exemplary additives are salts, for example sodium chloride (NaCl), graphite particles, and metallic and semiconductive particles. Such additives allow for improving the characteristics of the electrical spark or plasma formation when applying a high voltage to the tips of the electrodes.

The device may be handheld (e.g. a small handheld shock wave applicator - shock wave probe).

The device comprises a plurality of electrodes, i.e., at least two electrodes. The electrodes maybe spaced apart by a spark gap such that a spark can form between them when a high voltage as described above is applied. The electrodes may be adjustable to influence the lag time for the spark formation or compensate the distance for the erosion of the electrode tips. The electrodes may be adjustable by mechanical means which can be automated and motorized.

Preferably, the entire interior of the housing is potted. Thereby, all components held within the housing are securely held in place.

Preferably, at least a portion of the housing including the pulse pressure source is potted with the potting compound. Thereby, the generated pulses are dampened close to the location of their generation. This reduces the forces that must be absorbed by components held within the housing even further. In addition, noise of the shockwave generation may be reduced.

Preferably, the potting compound is optically transparent. This may enable manufacturing testing in the housing and may be particularly useful in the case of high-voltage testing for partial electrical discharges. In case of a potential malfunction this transparency is also preferable for the operator who can see the components and detect mechanical dislocations or unintended discharges.

Preferably, the device comprises at least one high-voltage cable. The high-voltage cable is potted with the potting compound. The high-voltage cable may supply the plurality of electrodes with electricity, i.e., with the necessary voltage to generate pulses. Potting the high-voltage cable provides the advantage that this electrical component is particularly well protected from vibrations caused by the pulse generation. Particularly preferred, the potting compound is electrically isolating. High-voltage as mentioned herein may mean voltages above 500 V or 1 kV. The cable may have a cable isolation in the potted part. However, if the potting compound is electrically isolating, the cable may not comprise a cable isolation inside of the housing. The potting therefore may protect the cable and the inner housing from humidity and access of ultrasound gel used for its acoustic coupling of the membrane to the patient's skin or other liquid during its operation and storage.

Preferably, the housing comprises at least a first housing part and a second housing part (also described as upper and lower housing parts herein). The housing parts are connected to each other with connection means, in particular mechanical connection means. The connection means may be one or more screws although any other connection means may also be suitable, e.g., glue, bolts, etc. The connection means extend into the interior of the housing and may be potted with the potting compound. Potting the connection means prevents loosening of the connection means by vibrations, e.g., vibrations caused by the pressure pulse source.

Preferably, at least a distal part and/or a proximal part of the housing is filled with the potting compound. More preferably, at least half of the interior of the housing is filled with the potting compound. Proximal and distal as used herein may refer to a perspective of an operator of the device. Proximal describes a direction that points towards the operator during the intended use, i.e., an operator holding the device, e.g., while applying shockwaves. Distal describes a direction that points away from the operator during intended use. Filling only a distal part may provide for dampening of the vibrations caused by the pulse generation and for protecting the pressure pulse source that may be arranged in a distal part of the housing. Filling only a proximal part may protect mechanical parts as well as electrical cables connecting to the plurality of electrodes.

Preferably, the housing comprises a first housing part and a second housing part, wherein a seal is provided between the first housing part and the second housing part. Thereby, potting the device may be simplified. In particular, the housing may be assembled (e.g., including all components) and then potted through a single opening or a plurality of openings in the housing. The seal may prevent a liquid potting compound from leaving the housing during a potting process. Further, the seal may protect the components as well as the potting compounds from external influences such as dirt and aggressive agents and processes such as sterilization.

Preferably, the seal is made of or comprises a hard or flexible material. The material is, in particular, silicone although other seal materials may also be used. If the material is flexible, this provides for a particularly good adjustment to the shapes of the housing parts and thus for a good isolation of the interior of the device.

Preferably, the seal extends around the pressure pulse source. The first housing part may be a first half of the housing and the second housing part may be a second half of the housing. The seal may be between these halves. The housing may have a longitudinally extending shape. The first and second housing parts may be separable along a longitudinal axis. The seal may extend in a plane parallel to a longitudinal axis of the housing. Thereby, a large opening for inserting the components inside of the housing is formed which simplifies manufacturing the device.

Preferably, the seal comprises a sleeve for receiving a high-voltage cable. The housing may be connected or connectable to a power and control unit with the high-voltage cable. The seal comprising a sleeve improves a seal inside of the housing as a seal between the housing, seal and cable is provided. The sleeve may surround the high-voltage cable partly or entirely.

Preferably, the sleeve is made of silicone.

Preferably, the first housing part and/or the second housing part comprises a groove for receiving the seal. This improves a sealing between the seal and the respective housing part. The groove may extend along an interface between the first and/or second housing part and the seal.

Preferably, the first housing part, the second housing part and the seal form an enclosed space. The enclosed space may include an opening for the shockwave opening and for a power connection. Thereby, the entire interior of the housing may be potted quickly by filling the enclosed space with the potting compound.

Preferably, the potting compound comprises or is at least one of a silicone, a polyurethane, an epoxy resin and an acrylic. The potting compound is preferably electrically isolating and/or preferably elastic. Further the potting compound may absorb vibrations.

Epoxy resins may be mixed with a hardener before use to initiate curing. The duration of the curing process is in the middle range and results in a thermoset with good temperature and chemical resistance. Epoxy resins are good electrical insulators.

Polyurethanes (also PUR or PU) may be processed into foams. They may be mixed with a hardener and harden quicker than epoxy resins. A special feature is that, depending on the composition, different densities and flexibilities can be achieved. PUR potting compounds are also relatively easy to remove. Some PUR potting compounds may require the surface to be treated with acid or mechanical abrasion before application to ensure ideal adhesion.

Silicones may be used as liquid resins or as curing resins in combination with a hardener. Silicones cure only very slowly but have extraordinary heat resistance and high mechanical flexibility and adhesion to surfaces. In addition, silicone potting compounds are relatively easy to remove. Silicones are good electrical insulators.

One example silicone is a polydimethylsiloxane with vinyl groups (and optionally further additives). One particular example is ELASTOSIL^{®} LR 3003/30 A/B (manufactured by Wacker Chemistry), which is a two-component compound. The two components are ELASTOSIL^{®} LR 3003/30 A and ELASTOSIL^{®} LR 3003/30 B.

Acrylic protective coatings for electronic components cure particularly quickly and have a very long shelf life. They may be transparent or glossy and can be easily sprayed on to protect electronics from corrosion and other damaging influences.

Preferably, the potting compound is at least one of: UV light curable, room temperature vulcanizing, a two-component potting compound and oxygen curable. Thereby, the potting compound may be inserted into the housing as a liquid and then hardened using any of the above-mentioned curing methods.

A further aspect of the present invention relates to a method of manufacturing a medical device for generating shockwaves. The device is preferably a device as described above. The method comprises the following steps:
- Providing a pressure pulse source comprising a chamber housing defining a chamber and a shockwave opening, the chamber being configured to be filled with a liquid, wherein a plurality of electrodes is disposed in the chamber and configured to be coupled to an electrical pulse-generation system, the plurality of electrodes including a first electrode and a second electrode, the first electrode and the second electrode defining a spark gap;
- Assembling a housing that surrounds at least a portion of the pressure pulse source; and
- Potting an interior of the housing at least partially, preferably entirely, with a potting compound configured to absorb vibrations caused by the generated shockwaves.

The invention will be described by way of example and with reference to the accompanying drawings in which:
Figure 1A is a perspective view of a shockwave applicator (shock wave probe) comprising a device according to the invention.
Figure 1B is a power and control unit for the shockwave applicator (shock wave probe) of figure 1A.
Figure 2 is an exploded view of a portion of a shockwave applicator (shock wave probe)
Figure 3A is a top view of the shockwave applicator (shock wave probe) shown in figure 2.
Figure 3B is a bottom view of the shockwave applicator (shock wave probe) shown in figures 2 and 3A.
Figure 3C is a longitudinal cross-section through the shockwave applicator (shock wave probe) shown in figures 2, 3A, and 3B.
Figure 4A shows a side view of a seal.
Figure 4B shows a perspective view of the seal of figure 4A.
Figure 5 is a further bottom view of the shockwave applicator probe (shock wave probe) during a potting process.

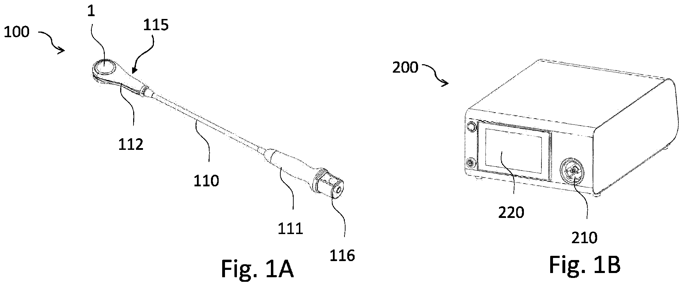

With reference to figures 1A and 1B, a portable hand-held shockwave applicator device 100 (also device for generating shockwaves herein) and a power and control unit 200 are illustrated (i.e., an acoustic pulse-generation system). The hand-held shockwave applicator (shock wave probe) device 100 includes a cable 110. One end of the cable 110 comprises a connector 111 and the other end of the cable 110 comprises a shockwave applicator (shock wave probe) 115 with a housing 112. Within the housing 112, a shockwave reflector 1 is arranged. The connector 111 can be connected to a corresponding female receptacle 210 of the power and control unit 200. The power and control unit 200 may also include a touchscreen 220 for operation of the power and control unit 200. The connector 111 includes a male end portion 116 that can be inserted into the female receptacle 210. The cable 110 may be a high voltage cable or rod and is preferably surrounded by an elastomeric insulation and a neutral electrical shielding braid.

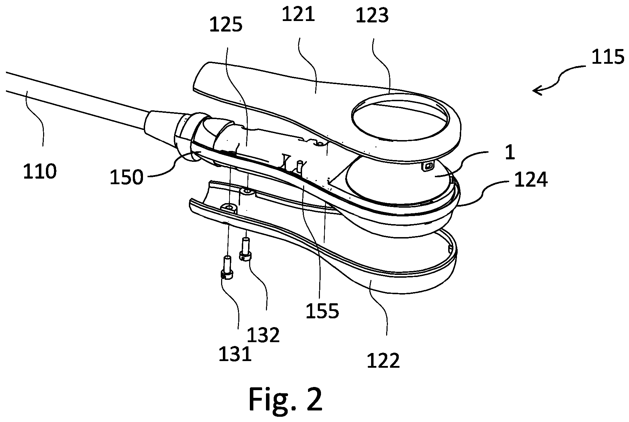

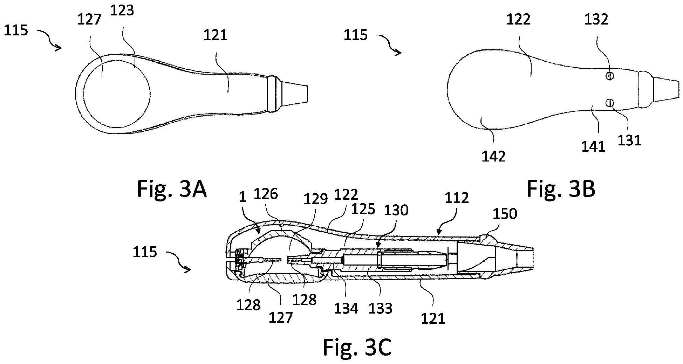

Figures 2 to 4B illustrate the shock wave probe 115 in further detail. As can be seen in figures 2, 3A, and 3B, the housing 112 comprises an upper housing part 121 and a lower housing part 122. In the shown embodiment, the upper housing part 121 has a rounded or curved shape. The lower housing part 122 also has a rounded or curved shape. Generally, the housing may be described as pear-shaped. The housing has a proximal part 141 with a cylindrical shape having a semi-circular cross-section. A distal part 142 has (roughly) a semispherical or semi-oval shape to accommodate a probe housing (see figures 2 and 3B). The upper housing part 121 includes a circular opening 123 (see figure 2 and 3A). The shockwave reflector 1 is arranged below the circular opening 123 and shockwaves are expelled through the circular opening 123. The upper housing part 121 and the lower housing part 122 may jointly form a closed shell and define a space encompassed therein. As can be seen, e.g., from figure 3C, the housing surrounds a pressure source comprising a probe housing 126 (also chamber housing herein). The probe housing 126 is closed by a membrane 127 and may be metallic. A chamber 129 having a closed volume formed by probe housing 126 and membrane 127 is filled with liquid. Electrodes 128 extend into the chamber 129. The tips of the electrodes 128 are gapped at a distance to facilitate a spark gap which creates the shockwave when energized. Briefly, when a high voltage is applied between the tips of the electrodes 128, a spark is formed and the shock wave is formed by the expanding plasma.. The shock wave has a nearly spherical amplitude distribution and therefore part of the shock wave front passes through the membrane 127 unreflected and followed shortly later by reflected portion of the shock wave. The reflection of the shock wave is achieved by the reflector 1 which is preferably made of metal which is acoustically denser than the liquid in chamber 129. The shock wave pulses generated within chamber 129 are transmitted through the membrane and thus towards a patient. The membrane 127 is acoustically coupled to the patient's skin by ultrasound gel or by a liquid like water.

Both, the upper housing part 121 and the lower housing part 122 extend from a distal end 124 of the shockwave applicator device 100 (see figure 2). Further the housing 112 is made of a rigid plastic material, e.g., polypropylene (PP).

Figure 3C further shows a connecting portion 130 of the shockwave generating device 1 to the cable 110. The connecting portion 130 may include elastomeric insulation bellows 133, a magnet, and a coil for moving the magnet. This may be used to adjust a distance between electrodes, i.e., to define a spark gap. Alternatively, the tips can be replaced with adjustable electrodes using other means such as piezo ceramics, magnets, motors with gear boxes, pneumatics, or hydraulics to change the tip distance. Further, the connecting portion 130 includes a high voltage cable 134 that connects cable 110 to the electrodes 128. The high voltage cable 134 may or may not include an additional insulation.

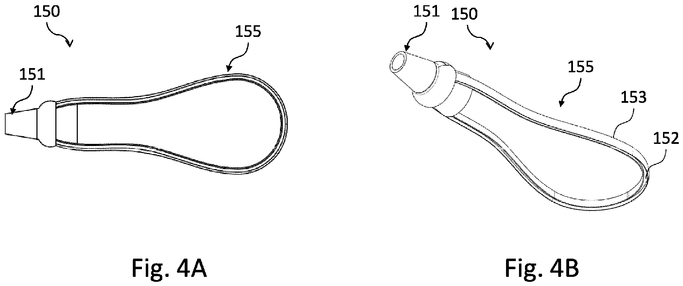

The device includes a seal 150 (see in particular figures 4A and 4B). The seal 150 is made of silicone. The seal 150 may include a sleeve 151 that surrounds the cable 110. A liquid tight seal is formed between cable 110 and sleeve 151. The seal 150 may also include a loop portion 155. The loop portion 155 extends around a circumference of the shock wave probe 115 (see figure 2). In the shown embodiment, the loop portion 155 is arranged between the upper housing part 121 and the lower housing part 122 which may not touch directly and may be separated by the loop portion 155. The loop portion 155 includes a top groove 152 and a bottom groove 153. The top groove 152 and bottom groove 153 each receive the lower housing part 122 and the upper housing part 121 respectively. The upper housing part 121 and the lower housing part 122 may include notches extending along an edge portion of the upper housing part 121 and/or the lower housing part 122 that engage the top groove 152 and the bottom groove 153. The upper housing part 121 and the lower housing part 122 may be fixated to each other using screws 131 and 132 (see figures 2 and 3B). The screws 131 and 132 push the upper housing part 121 and lower housing part 122 towards each other and compress the seal 150 between them. Thereby, a liquid tight seal is provided between upper housing part 121 and lower housing part 122.

As can be seen from figures 2 and 3C, the space encompassed by the housing 112 is filled with a potting compound 125. The potting compound 125 fills the space encompassed by the housing 112 entirely and fixates all components within the housing 112 in place. Thus, the potting compound 125 secures the probe housing 126, electrodes 128, screws 131, 132, and connecting portion 130, 133 in place inside the housing. The potting compound dampens vibrations that may travel through the housing, in particular vibrations caused by a generation of a pressure pulse. The potting compound also serves as additional electrical insulation. In this example, the potting compound is silicone.

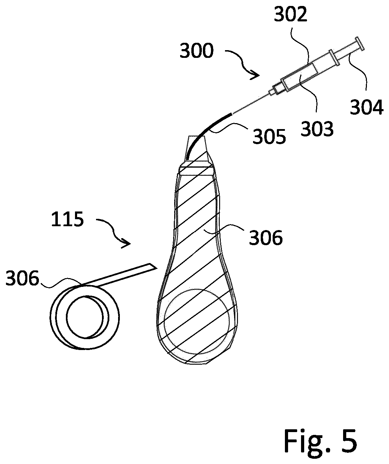

Figure 5 illustrates how the potting compound may be added when manufacturing a shock wave probe 115. The shock wave probe 115 is assembled as usual, i.e., the probe housing 126, betwen upper housing part 121 and lower housing part 122, the electrodes 128, and the connecting portion 130, using screws 131 and 132 for the fixation of the parts, are assembled. These components are housed within the housing 112 which is sealed with seal 150. Then, after assembly of the shock wave probe 115, a potting compound is inserted using insertion system 300. The insertion system comprises a tube 301 and a syringe 302 filled with a liquid potting compound 303.

The housing may include a potting insertion opening, to which the tube 301 may be connected. The insertion opening may be provided in a proximal part of the housing as illustrated in figure 5, though it is also possible to provide the opening in a distal part. The syringe 302 may be filled with the liquid potting compound 303. The liquid potting compound 303 is pushed from the syringe 302 (e.g., using pusher rod 304) through the tube 301 into the potting insertion opening of the housing 112. The opening may be part of the upper or lower housing part 121, 122 or may be provided in between the upper housing part 121 and lower housing part 122. Preferably, the lower housing part 122 comprises the opening. The upper housing part 121 or the lower housing part 122 may additionally comprise a ventilation opening that allows air to escape while the housing is filled with the liquid potting compound.

In addition, as illustrated by the hatching in figure 5, the assembled probe may additionally be tape wrapped with a tape 306, e.g. Scotch tape, while filling the space enclosed by the housing with the liquid potting compound. Alternatively, to the tape a mechanical and removable potting form can be used. Silicone resin may have a low viscosity and may require high pressures of up to 10 bar (6-7 bar in some examples) when injecting the silicone resin into the housing. The tape wrapping may provide an additional sealing. The silicone resin is based on a two-component system and does not require any additional curing. The two-component system comprises a base material and a curing agent (or catalyst) and may harden about 12 h after mixing. The liquid base material and the liquid curing agent may be mixed prior to a loading in the syringe 302.

## Claims

1. Medical device for generating shockwaves (115) comprising a housing (112), the housing encompassing:
a pressure pulse source comprising a chamber housing (126) defining a chamber (129) and a shockwave opening, the chamber (129) being configured to be filled with a liquid; and
a plurality of electrodes (128) disposed in the chamber (129) and configured to be coupled to an electrical pulse-generation system, the plurality of electrodes (128) including a first electrode and a second electrode, the first electrode and the second electrode defining a spark gap;
wherein at least a part of an interior of the housing (112) is potted with a potting compound (125) configured to absorb vibrations caused by the generated shockwaves.

2. Device according to claim 1, wherein the entire interior of the housing (112) is potted.

3. Device according to claim 1 or 2, wherein at least a portion of the housing (112) including the pressure pulse source is potted with the potting compound (125).

4. Device according to any one of the previous claims, wherein the device comprises at least one high-voltage cable and wherein the high voltage cable (134) is potted with the potting compound (125).

5. Device according to any one of the previous claims, wherein the housing (112) comprises at least a first housing part and a second housing part, wherein the housing parts (121, 122) are connected to each other with connection means, preferably one or more screws (131, 132), and wherein the connection means extend into an interior of the housing (112) and wherein the connection means are potted with the potting compound (125).

6. Device according to any one of the previous claims, wherein the device comprises a membrane (127) for transmitting the shockwaves, the membrane (127) defining a portion of the chamber (129), wherein the potting compound (125) fixates the membrane (127).

7. Device according to any one of claims 1 to 4 and 6, wherein the housing (112) comprises a first housing part and a second housing part, wherein a seal (150) is provided between the first housing part and the second housing part.

8. Device according to claim 7, wherein the seal (150) is made of or comprises a hard or flexible material, in particular silicone.

9. Device according to one of claims 7 or 8, wherein the seal (150) comprises a sleeve (151) for receiving a high voltage cable (134).

10. Device according to one of claims 7 to 9, wherein the first housing part and/or the second housing part comprise(s) a groove for receiving the seal (150).

11. Device according to one of claims 7 to 10, wherein the first housing part, the second housing part and the seal (150) form an enclosed space, the enclosed space preferably including an opening for the shockwave opening and for a power connection.

12. Device according to any one of the previous claims, wherein the potting compound (125) comprises or is at least one of: a silicone, a polyurethane, an epoxy resin, and an acrylic.

13. Device according to any one of the previous claims, wherein the potting compound (125) is at least one of: UV light curable, room-temperature vulcanizing, a two-component potting compound and oxygen curable.

14. Device according to any one of the previous claims, wherein the device is sterilizable using steam, Gamma rays, ethylene oxide and/or formaldehyde.

15. Method of manufacturing a medical device for generating shockwaves, preferably a device according to any of the preceding claims, comprising the following steps:
- Providing a pressure pulse source comprising a chamber housing defining a chamber (129) and a shockwave opening, the chamber (129) being configured to be filled with a liquid, wherein a plurality of electrodes (128) is disposed in the chamber (129) and configured to be coupled to an electrical pulse-generation system, the plurality of electrodes (128) including a first electrode and a second electrode, the first electrode and the second electrode defining a spark gap;
- Assembling a housing (112) that surrounds at least a portion of the pressure pulse source; and
- Potting an interior of the housing (112) at least partially, preferably entirely, with a potting compound (125) configured to absorb vibrations caused by the generated shockwaves.

## Patentansprüche

1. Medizinische Vorrichtung zur Erzeugung von Stoßwellen (115), die ein Gehäuse (112) aufweist, wobei das Gehäuse umfasst:
eine Druckimpulsquelle, die ein Kammergehäuse (126) aufweist, das eine Kammer (129) und eine Stoßwellenöffnung definiert, wobei die Kammer (129) konfiguriert, mit einer Flüssigkeit gefüllt zu werden; und
mehrere Elektroden (128), die in der Kammer (129) angeordnet und konfiguriert sind, mit einem elektrischen Impulserzeugungssystem gekoppelt zu werden, wobei die mehreren Elektroden (128) eine erste Elektrode und eine zweite Elektrode umfassen, wobei die erste Elektrode und die zweite Elektrode eine Funkenstrecke definieren;
wobei zumindest ein Teil eines Innenraums des Gehäuses (112) mit einer Vergussmasse (125) vergossen ist, die konfiguriert ist, durch die erzeugten Stoßwellen verursachte Vibrationen zu absorbieren.

2. Vorrichtung nach Anspruch 1, wobei das gesamte Innere des Gehäuses (112) vergossen ist.

3. Vorrichtung nach Anspruch 1 oder 2, wobei zumindest ein Teil des Gehäuses (112), der die Druckimpulsquelle enthält, mit der Vergussmasse (125) vergossen ist.

4. Vorrichtung nach einem der vorhergehenden Ansprüche, wobei die Vorrichtung mindestens ein Hochspannungskabel aufweist und wobei das Hochspannungskabel (134) mit der Vergussmasse (125) vergossen ist.

5. Vorrichtung nach einem der vorhergehenden Ansprüche, wobei das Gehäuse (112) mindestens einen ersten Gehäuseteil und einen zweiten Gehäuseteil aufweist, wobei die Gehäuseteile (121, 122) mit Verbindungseinrichtungen, vorzugsweise einer oder mehreren Schrauben (131, 132), miteinander verbunden sind, und wobei sich die Verbindungseinrichtungen in ein Inneres des Gehäuses (112) erstrecken und wobei die Verbindungseinrichtungen mit der Vergussmasse (125) vergossen sind.

6. Vorrichtung nach einem der vorhergehenden Ansprüche, wobei die Vorrichtung eine Membran (127) zum Übertragen der Stoßwellen aufweist, wobei die Membran (127) einen Teil der Kammer (129) definiert, wobei die Vergussmasse (125) die Membran (127) fixiert.

7. Vorrichtung nach einem der Ansprüche 1 bis 4 und 6, wobei das Gehäuse (112) einen ersten Gehäuseteil und einen zweiten Gehäuseteil aufweist, wobei eine Dichtung (150) zwischen dem ersten Gehäuseteil und dem zweiten Gehäuseteil vorgesehen ist.

8. Vorrichtung nach Anspruch 7, wobei die Dichtung (150) aus einem harten oder flexiblen Material, insbesondere aus Silikon, besteht oder dieses aufweist.

9. Vorrichtung nach einem der Ansprüche 7 oder 8, wobei die Dichtung (150) eine Hülse (151) zur Aufnahme eines Hochspannungskabels (134) aufweist.

10. Vorrichtung nach einem der Ansprüche 7 bis 9, wobei der erste Gehäuseteil und/oder der zweite Gehäuseteil eine Nut zur Aufnahme der Dichtung (150) aufweist.

11. Vorrichtung nach einem der Ansprüche 7 bis 10, wobei der erste Gehäuseteil, der zweite Gehäuseteil und die Dichtung (150) einen umschlossenen Raum bilden, wobei der umschlossene Raum vorzugsweise eine Öffnung für die Stoßwellenöffnung und für einen Stromanschluss aufweist.

12. Vorrichtung nach einem der vorhergehenden Ansprüche, wobei die Vergussmasse (125) mindestens eines aufweist von oder besteht aus: einem Silikon, einem Polyurethan, einem Epoxidharz und einem Acryl.

13. Vorrichtung nach einem der vorhergehenden Ansprüche, wobei die Vergussmasse (125) mindestens eines ist von: UV-Licht härtend, bei Raumtemperatur vulkanisierend, eine Zweikomponenten-Vergussmasse und sauerstoffhärtend.

14. Vorrichtung nach einem der vorhergehenden Ansprüche, wobei die Vorrichtung unter Verwendung von Dampf, Gammastrahlen, Ethylenoxid und/oder Formaldehyd sterilisierbar ist.

15. Verfahren zur Herstellung einer medizinischen Vorrichtung zur Erzeugung von Stoßwellen, vorzugsweise einer Vorrichtung nach einem der vorhergehenden Ansprüche, das die folgenden Schritte aufweist:
- Bereitstellen einer Druckimpulsquelle, die ein Kammergehäuse aufweist, das eine Kammer (129) und eine Stoßwellenöffnung definiert, wobei die Kammer (129) konfiguriert ist, mit einer Flüssigkeit gefüllt zu werden, wobei mehrere Elektroden (128) in der Kammer (129) angeordnet ist und konfiguriert sind, mit einem elektrischen Impulserzeugungssystem gekoppelt zu werden, wobei die mehreren Elektroden (128) eine erste Elektrode und eine zweite Elektrode umfassen, wobei die erste Elektrode und die zweite Elektrode eine Funkenstrecke definieren;
- Montieren eines Gehäuses (112), das zumindest einen Abschnitt der Druckimpulsquelle umgibt; und
- Vergießen eines Innenraums des Gehäuses (112) zumindest teilweise, vorzugsweise vollständig, mit einer Vergussmasse (125), die konfiguriert ist, durch die erzeugten Stoßwellen verursachte Vibrationen zu absorbieren.

## Revendications

1. Dispositif médical de génération d'ondes de choc (115) comprenant un boîtier (112), le boîtier comprenant :
une source d'impulsions de pression comprenant un boîtier de chambre (126) définissant une chambre (129) et une ouverture pour ondes de choc, la chambre (129) étant configurée pour être remplie d'un liquide ; et
une pluralité d'électrodes (128) disposées dans la chambre (129) et configurées pour être couplées à un système de génération d'impulsions électriques, la pluralité d'électrodes (128) comprenant une première électrode et une deuxième électrode, la première électrode et la deuxième électrode définissant un éclateur ;
dans lequel au moins une partie de l'intérieur du boîtier (112) est enrobée d'un composé d'enrobage (125) configuré pour absorber les vibrations causées par les ondes de choc générées.

2. Dispositif selon la revendication 1, dans lequel tout l'intérieur du boîtier (112) est enrobé.

3. Dispositif selon l'une des revendications 1 et 2, dans lequel au moins une partie du boîtier (112) comprenant la source d'impulsions de pression est enrobée avec le composé d'enrobage (125).

4. Dispositif selon l'une quelconque des revendications précédentes, dans lequel le dispositif comprend au moins un câble haute tension et dans lequel le câble haute tension (134) est enrobé avec le composé d'enrobage (125).

5. Dispositif selon l'une quelconque des revendications précédentes, dans lequel le boîtier (112) comprend au moins une première partie de boîtier et une deuxième partie de boîtier, dans lequel les parties de boîtier (121, 122) sont reliées l'une à l'autre par des moyens de connexion, de préférence une ou plusieurs vis (131, 132), et dans lequel les moyens de connexion s'étendent à un intérieur du boîtier (112) et dans lequel les moyens de connexion sont enrobés avec le composé d'enrobage (125).

6. Dispositif selon l'une quelconque des revendications précédentes, dans lequel le dispositif comprend une membrane (127) pour transmettre les ondes de choc, la membrane (127) définissant une partie de la chambre (129), dans lequel le composé d'enrobage (125) fixe la membrane (127).

7. Dispositif selon l'une quelconque des revendications 1 à 4 et 6, dans lequel le boîtier (112) comprend une première partie de boîtier et une deuxième partie de boîtier, dans lequel un joint d'étanchéité (150) est prévu entre la première partie de boîtier et la deuxième partie de boîtier.

8. Dispositif selon la revendication 7, dans lequel le joint d'étanchéité (150) est constitué d'un matériau dur ou souple, en particulier du silicone, ou comprend un tel matériau.

9. Dispositif selon l'une des revendications 7 et 8, dans lequel le joint d'étanchéité (150) comprend un manchon (151) destiné à recevoir un câble haute tension (134).

10. Dispositif selon l'une des revendications 7 à 9, dans lequel la première partie de boîtier et/ou la deuxième partie de boîtier comprennent une rainure destinée à recevoir le joint d'étanchéité (150).

11. Dispositif selon l'une des revendications 7 à 10, dans lequel la première partie de boîtier, la deuxième partie de boîtier et le joint d'étanchéité (150) forment un espace clos, l'espace clos comprenant de préférence une ouverture pour l'ouverture pour ondes de choc et pour une connexion électrique.

12. Dispositif selon l'une quelconque des revendications précédentes, dans lequel le composé d'enrobage (125) comprend ou est au moins l'un des composés suivants : un silicone, un polyuréthane, une résine époxy et un acrylique.

13. Dispositif selon l'une quelconque des revendications précédentes, dans lequel le composé d'enrobage (125) est au moins l'un des suivants : durcissable aux UV, vulcanisable à température ambiante, un composé d'enrobage à deux composants et durcissable à l'oxygène.

14. Dispositif selon l'une quelconque des revendications précédentes, le dispositif étant stérilisable à la vapeur, aux rayons gamma, à l'oxyde d'éthylène et/ou au formaldéhyde.

15. Procédé de fabrication d'un dispositif médical de génération d'ondes de choc, de préférence d'un dispositif selon l'une quelconque des revendications précédentes, comprenant les étapes suivantes :
- Fournir une source d'impulsions de pression comprenant un boîtier de chambre définissant une chambre (129) et une ouverture pour ondes de choc, la chambre (129) étant configurée pour être remplie d'un liquide, une pluralité d'électrodes (128) étant disposées dans la chambre (129) et configurées pour être couplées à un système de génération d'impulsions électriques, la pluralité d'électrodes (128) comprenant une première électrode et une deuxième électrode, la première électrode et la deuxième électrode définissant un éclateur ;
- Assembler un boîtier (112) qui entoure au moins une partie de la source d'impulsions de pression ; et
- Enrober un intérieur du boîtier (112) au moins partiellement, de préférence entièrement, avec un composé d'enrobage (125) configuré pour absorber les vibrations causées par les ondes de choc générées.
